# EUROPÄISCHE PATENTANMELDUNG

(11) **EP 1 418 237 A2**
(43) Veröffentlichungstag der Anmeldung: **12.05.2004**
(21) Anmeldenummer: 03022590.8
(22) Anmeldetag: 06.10.2003
(51) Int. Cl.: C12N 15/55, C12N 9/20, C12N 1/21, C12P 7/22, C12P 7/62, C12P 7/64, C12P 41/00, A61K 31/045, A61K 31/21

(54) **Anti-Kazlauskas-Lipasen**

(30) Priorität: 16.10.2002 DE 10248166
(71) Anmelder: Bayer Chemicals AG, 51368 Leverkusen (DE)
(72) Erfinder: Bosch, Boris, Dr., 50668 Köln (DE); Meissner, Ruth, Dr., 51375 Leverkusen (DE); Berendes, Frank, Dr., 48151 Münster (DE); Koch, Rainhard, Dr., 51065 Köln (DE)

(57) **Zusammenfassung**

Die vorliegende Erfindung betrifft Polypeptide, die biologische Aktivität von Lipasen ausüben und eine der Kazlauskas-Regel entgegengesetzte Selektivität aufweisen, Wirtszellen enthaltend Nukleinsäuren, die für diese Polypeptide codieren, die Nukleinsäuren selbst sowie die zur Herstellung der erfindungsgemäßen Wirtszellen benötigten Vektoren. In einem weiteren Aspekt betrifft die Erfindung ein Verfahren zur Herstellung von enantiomerenangereicherten Estern und Alkoholen, das vorteilhafterweise in Gegenwart eines Racemisierungskatalysator durchgeführt wird.

## Beschreibung

Die vorliegende Erfindung betrifft Polypeptide, die die biologische Aktivität von Lipasen ausüben und eine der Kazlauskas-Regel entgegengesetzte Selektivität aufweisen, Wirtszellen enthaltend Nukleinsäuren, die für diese Polypeptide codieren, die Nukleinsäuren selbst, sowie die zur Herstellung der erfindungsgemäßen Wirtszellen benötigten Vektoren. In einem weiteren Aspekt betrifft die Erfindung ein Verfahren zur Herstellung von enantiomerenangereicherten Estern und Alkoholen, das Vorteilhafterweise in Gegenwart eines Racemisierungskatalysators durchgeführt wird.

Enantiomerenangereicherte Alkohole sind wertvolle Verbindungen zur Herstellung von Agrochemikalien, Arzneimitteln, Flüssigkristallen oder Zwischenprodukten davon. Ihre Herstellung erfolgt häufig unter Verwendung chiraler Übergangsmetallkatalysatoren. Dabei sind insbesondere Reduktionen von Ketonen durch Transferhydrierung und Hydrierung zu nennen (siehe auch Noyori: Asymmetric Catalysis in Organic Synthesis, J. Wiley, 1994). Problematisch an den chemisch katalytischen Verfahren sind die oft hohen Kosten der Katalysatoren, schwankende Edelmetallpreise und hohe Anforderungen an die Reinheit der Substrate.

Die enzymatische kinetische Racematspaltung von sekundären Alkoholen durch Transesterifizierung und ihre reversible Reaktion, die enzymatische Hydrolyse von Estern sekundärer Alkohole stellen weitere wichtige, industriell nutzbare Verfahren zur Herstellung enantiomerenangereicherter Alkohole dar. Dabei wird jeweils ein Enantiomer des racemischen Alkohols in Gegenwart eines geeigneten Acyldonors selektiv in den entsprechenden Ester überführt bzw. jeweils ein Enantiomer eines racemischen Esters selektiv durch Hydrolyse der Esterbindung in den entsprechenden Alkohol überführt. Die Transesterifizierung kann bedingt durch die oft gute Löslichkeit der Substrate häufig in organischen Lösungsmitteln und hohen Konzentrationen durchgeführt werden. Weiterhin sind racemische Gemische von Alkoholen in der Regel leichter erhältlich als racemische Ester. Aus diesen Gründen ist die enzymkatalysierte Transesterifizierung gegenüber der enzymkatalysierten hydrolytischen Spaltung von Estern zumeist bevorzugt.

Inhärente Nachteile sind jedoch die maximal erreichbare Ausbeute von 50 % und die Tatsache, dass der Enantiomerenüberschuss in Abhängigkeit der Enzymselektivität mit steigendem Umsatz stark abnehmen kann. Ein weiterer Nachteil ist, dass man nach der sogenannten Kazlauskas-Regel (Kazlauskas et al., J. Org. Chem. 1991, 56, 2656) mit den bisher bekannten Enzymen aufgrund der speziellen Strukturen der aktiven Zentren eine bestimmte, vorhersagbare räumliche Struktur der Produkte erhält.

Demnach läuft die enzymkatalysierte Transesterifizierung eines racemischen sekundären Alkohols üblicherweise gemäß folgendem Schema ab:

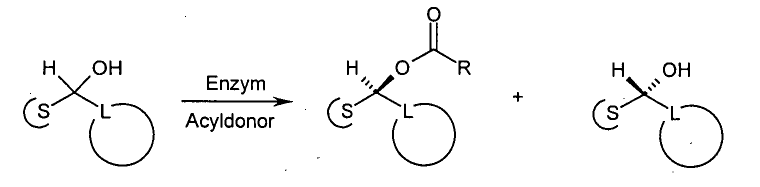

Hierin steht S für einen relativ kleinen und L für einen relativ großen Substituenten. Die Abbildung zeigt die absolute Stereochemie. Für die Priorisierung von S und L und die daraus folgende Bestimmung der absoluten Konfiguration (R oder S) gilt die Cahn-Ingold-Prelog-Regel (CIP-Regel, siehe auch J. March, Advanced Organic Chemistry, 4. Ed., 1992, Wiley Interscience, S. 109-115).

Darüber hinaus besagt die Kazlauskas-Regel, dass Enzyme, wie insbesondere Lipasen, dann besonders stereoselektiv arbeiten, wenn der räumliche Anspruch der Substituenten S und L sehr unterschiedlich ist.

Es bestand daher das Bedürfnis, Polypeptide bereitzustellen, die die Transesterifizierung von chiralen sekundären Alkoholen zu den entsprechenden Estern mit entgegengesetzter Selektivität bezüglich der Kazlauskas-Regel mit guten Enantioselektivitäten ermöglichen. Solche Enzyme werden im Folgenden Anti-Kazlauskas-Lipasen genannt.

Es wurden nun Polypeptide gefunden, die die biologische Aktivität einer Anti-Kazlauskas-Lipase ausüben und eine Aminosäuresequenz umfassen, die eine zumindest 60 %-ige, vorzugsweise mindestens 80 %-ige, besonders bevorzugt mindestens 90 %-ige und ganz besonders bevorzugt mindestens 95 %-ige Identität mit einer Aminosäuresequenz gemäß SEQ ID NO: 2 über den Ausschnitt von Aminosäure 110 bis Aminosäure 280 und vorzugsweise über deren Gesamtlänge aufweist. Eine katalytische Triade wird vermutlich durch die Aminosäuren 118, 243 und 271 gemäß SEQ ID NO: 2 gebildet.

Der Ausdruck "Polypeptide", wie er im Rahmen der Erfindung verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch bekannte chemische Verfahren modifiziert sein können. Solche Modifikationen können an verschiedenen Stellen und mehrfach in einem Polypeptid vorkommen, wie beispielsweise an dem Peptid-Rückgrat, an der Aminosäure-Seitenkette, am Aminound/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form reifer Proteine oder als Teile größerer Proteine, z.B. als Fusionsproteine, vorliegen. Weiterhin können sie Sezernierungs- oder Leader-Sequenzen, Pro-Sequenzen, Sequenzen, die eine einfache Reinigung ermöglichen, wie mehrfache Histidin-Reste, oder zusätzliche stabilisierende Aminosäuren aufweisen.

Die erfindungsgemäßen Polypeptide müssen nicht vollständige Anti-Kazlauskas-Lipasen darstellen, sondern können auch nur Fragmente davon sein, solange sie zumindest noch 50 % der biologischen Aktivität der vollständigen Anti-Kazlauskas-Lipase aufweisen. Polypeptide, die zumindest 50 % der biologischen Aktivität der Anti-Kazlauskas-Lipase mit einer Aminosäuresequenz gemäß SEQ ID NO: 2 ausüben, sind ebenfalls von der Erfindung umfasst.

Die erfindungsgemäßen Polypeptide können weiterhin im Vergleich zu der entsprechenden Region von natürlichen Anti-Kazlauskas-Lipasen Deletionen oder Aminosäuresubstitutionen aufweisen, solange sie zumindest noch eine biologische Aktivität der vollständigen Anti-Kazlauskas-Lipase ausüben. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| Ursprünglicher Rest | Substitution |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Der Grad der Identität der Aminosäuresequenzen wird vorzugsweise bestimmt mit Hilfe des Programms BLASTP + BEAUTY Version 2.0.4. (Altschul et al., Nucleic Acids Res., 25, 1997 3389-3402).

Ein besonders bevorzugtes Polypeptid ist die Anti-Kazlauskas-Lipase mit der Aminosäuresequenz gemäß SEQ ID NO: 2.

Der Ausdruck "biologische Aktivität einer vollständigen Anti-Kazlauskas-Lipase", wie er im Rahmen der Erfindung verwendet wird, bedeutet die Fähigkeit zur Umsetzung von Substraten entgegen der Kazlauskas-Regel.

Von der Erfindung sind weiterhin Nukleinsäuren umfasst. Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, oder cDNA.

Besonders bevorzugt umfassen die erfindungsgemäßen Nukleinsäuren eine Sequenz ausgewählt aus
a) der Sequenz gemäß SEQ ID NO: 1,
b) Sequenzen, die für ein Polypeptid codieren, welches die Aminosäuresequenz gemäß SEQ ID NO: 2 umfasst,
c) zumindest 14 Basenpaare langen Teilsequenzen der unter a) oder b) definierten Sequenzen,
d) Sequenzen, welche an die unter a) oder b) definierten Sequenzen hybridisieren,
e) Sequenzen, welche eine zumindest 70 %ige, bevorzugt eine 85 %ige, besonders bevorzugt eine 90 %ige Identität mit den unter a) definierten Sequenzen aufweisen,
f) Sequenzen, welche eine zumindest 70 %ige, bevorzugt eine 80 %ige, besonders bevorzugt eine 90 %ige Identität der unter b) definierten Sequenzen aufweisen,
g) Sequenzen, welche zu den unter a) oder b) definierten Sequenzen komplementär sind, und
h) Sequenzen, welche aufgrund der Degeneriertheit des genetischen Codes für dieselbe Aminosäuresequenz codieren wie die unter a) bis f) definierten Sequenzen.

Eine ganz besonders bevorzugte Nukleinsäure ist ein DNA-Molekül mit der Sequenz gemäß SEQ ID NO: 1.

Der Ausdruck "hybridisieren", wie er im Rahmen der Erfindung verwendet wird, beschreibt den Vorgang, bei welchem ein einzelsträngiges Nukleinsäuremolekül mit einem komplementären Strang eine Basenpaarung eingeht. Auf diese Weise können ausgehend von der im Rahmen der Erfindung offenbarten Sequenzinformation beispielsweise DNA-Fragmente aus anderen Bakterien isoliert werden, welche für ein Polypeptid kodieren, das zumindest 50 % der Aktivität des erfindungsgemäßen Polypeptids mit der Aminosäuresequenz gemäß SEQ ID NO: 2 aufweist.

Hybridisierungsbedingungen werden nach folgender Formel näherungsweise berechnet:

Die Schmelztemperatur Tm = 81.5°C + 16.6 log{c(Na⁺)] + 0.41(%G + C)) - 500/n (Lottspeich und Zorbas, 1998, Bioanalytik, Spektrum Akademischer Verlag, Heidelberg, Berlin).

Dabei ist c die Konzentration und n die Länge des hybridisierenden Sequenzabschnitts in Basenpaaren. Für eine Sequenz >100 bp entfällt der Ausdruck 500/n. Mit höchster Stringenz wird bei einer Temperatur 5 bis 15°C unterhalb Tm und einer Ionenstärke von 15 mM Na⁺ (entspricht 0.1 x SSC) gewaschen. Wird eine RNA-Probe zur Hybridisierung verwendet, so ist der Schmelzpunkt um 10 bis 15°C höher.

Bevorzugte Hybridisierungsbedingungen sind nachstehend angegeben:
Hybridisierungslösung: DIG Easy Hyb (Fa.: Roche)
Hybridisierungstemperatur: 37°C, bevorzugt 42°C (DNA-DNA), 50°C (DNA-RNA).
   1. Waschschritt: 2mal SSC, 2 mal 5 min bei Raumtemperatur;
   2. Waschschritt: 2 mal 15min in 1X SSC, bei 50°C; bevorzugt 0,5X SSC, bei 65°C; besonders bevorzugt 0,2X SSC, bei 65°C.

Der Grad der Identität der Nukleinsäuren wird vorzugsweise bestimmt mit Hilfe des Programms NCBI BLASTN Version 2.0.4..

Gegenstand der vorliegenden Erfindung sind weiterhin DNA-Konstrukte, die eine erfindungsgemäße Nukleinsäure und einen heterologen Promotor umfassen.

Der Ausdruck "heterologer Promotor", wie er im Rahmen der Erfindung verwendet wird, bezieht sich auf einen Promotor, der andere Eigenschaften als derjenige Promotor aufweist, der im Ursprungsorganismus die Expression des betreffenden Gens kontrolliert.

Die Auswahl von heterologen Promotoren ist davon abhängig, ob zur Expression pro- oder eukaryotische Zellen oder zellfreie Systeme verwendet werden. Beispiele für heterologe Promotoren sind der 35S Promoter des Blumenkohlmosaikvirus für pflanzliche Zellen, der Promoter der Alkoholdehydrogenase für Hefezellen, die LacZ-, T3-, T7- oder SP6-Promotoren für prokaryotische Zellen oder zellfreie Systeme.

Gegenstand der vorliegenden Erfindung sind ferner Vektoren, die eine erfindungsgemäße Nukleinsäure oder ein erfindungsgemäßes DNA-Konstrukt enthalten. Als Vektoren können alle in molekularbiologischen Laboratorien verwendete Phagen, Plasmide, Phaghmide, Phasmide, Cosmide, YACs, BACs, künstliche Chromosomen oder Partikel, die für einen Partikelbeschuss geeignet sind, verwendet werden.

Bevorzugte Vektoren sind zum Beispiel die p4XXprom. Vektorserie für Hefezellen, pSPORT-Vektoren (Fa. Invitrogen) für bakterielle Zellen, lamdaZAP (Fa. Stratagene) für Phagen oder den Gateway Vektoren (Fa. Invitrogen) für verschiedene Expressionssysteme in bakteriellen Zellen oder Baculovirus.

Gegenstand der vorliegenden Erfindung sind weiterhin Wirtszellen, die eine erfindungsgemäße Nukleinsäure, ein erfindungsgemäßes DNA-Konstrukt oder einen erfindungsgemäßen Vektor enthalten.

Der Ausdruck "Wirtszelle", wie er im Rahmen der Erfindung verwendet wird, bezieht sich auf Zellen, die natürlicherweise die erfindungsgemäßen Nukleinsäuren nicht enthalten.

Als Wirtszellen eignen sich sowohl prokaryotische Zellen, vorzugsweise *E. coli*, als auch eukaryotische Zellen, wie Zellen von Saccharomyces cerevisiae, Pichia pastoris, Insekten, Pflanzen, Froschoozyten und Zelllinien von Säugern.

Die erfindungsgemäßen Nukleinsäuren können auf die übliche Weise hergestellt werden. Beispielsweise können die Nukleinsäuremoleküle vollständig chemisch synthetisiert werden. Man kann auch kurze Stücke der erfindungsgemäßen Nukleinsäuren chemisch synthetisieren und solche Oligonukleotide radioaktiv oder mit einem Fluoreszenzfarbstoff markieren. Die markierten Oligonukleotide können dann zum Beispiel verwendet werden, um ausgehend von Pflanzen-mRNA hergestellte cDNA-Banken zu durchsuchen. Klone, an die die markierten Oliogonukleotide hybridisieren, werden zur Isolierung der betreffenden DNA-Fragmente selektiert. Nach der Charakterisierung der isolierten DNA erhält man auf einfache Weise die erfindungsgemäßen Nukleinsäuren.

Die erfindungsgemäße Nukleinsäuren können auch mittels PCR-Verfahren unter Verwendung chemisch synthetisierter Oligonukleotide hergestellt werden.

Der Ausdruck "Oligonukleotid(e)", wie er im Rahmen der Erfindung verwendet wird, bedeutet DNA-Moleküle, die aus 10 bis 50 Nukleotiden, vorzugsweise 15 bis 30 Nukleotiden, bestehen. Sie werden chemisch synthetisiert und können als Sonden verwendet werden.

Die erfindungsgemäßen Polypeptide können auf verschiedenem Wege gewonnen werden, z.B. durch chemische Methoden wie der Festphasenmethode. Zur Gewinnung größerer Proteinmengen empfiehlt sich die Verwendung rekombinanter Methoden. Die Expression eines klonierten Anti-Kazlauskas-Lipase Gens oder Fragmente davon kann in an sich bekannter Weise in einer Reihe von passenden Wirtszellen erfolgen. Zu diesem Zweck wird beispielsweise eine erfindungsgemäße Nukleinsäure mit Hilfe bekannter Methoden in eine Wirtszelle eingeführt.

Die Integration des klonierten Anti-Kazlauskas-Lipase Gens die Wirtszelle liegt im Umfang der vorliegenden Erfindung. Vorzugsweise wird das Gen oder Fragmente davon in ein Plasmid gebracht, und die kodierenden Regionen des Anti-Kazlauskas-Lipase Gens oder Fragmente davon mit einem konstitutiven oder induzierbaren Promotor funktionell verknüpft.

Die grundlegenden Schritte zur Herstellung der rekombinanten Anti-Kazlauskas-Lipase sind:
1. Gewinnung einer natürlichen, synthetischen oder semi-synthetischen DNA, die für die Anti-Kazlauskas-Lipase kodiert.
2. Einbringen dieser DNA in einen Expressionsvektor, der geeignet ist, die Anti-Kazlauskas-Lipase zu exprimieren, entweder alleine oder als Fusionsprotein.
3. Transformation einer passenden, vorzugsweise prokaryontischen Wirtszelle mit diesem Expressionsvektor.
4. Anzucht dieser transformierten Wirtszelle in einer Weise, die geeignet ist, die Anti-Kazlauskas-Lipase zu exprimieren.
5. Ernte der Zellen und Aufreinigung Anti-Kazlauskas-Lipase durch geeignete, bekannte Methoden.

Die kodierenden Regionen der Anti-Kazlauskas-Lipase können dabei mit den üblichen Methoden in *E. coli* exprimiert werden. Geeignete Expressionssysteme für *E. coli* sind kommerziell erhältlich, so die Expressionsvektoren der pET-Serie, z.B. pET3a, pET23a, pET28a mit His-Tag oder pET32a mit His-Tag zur einfachen Aufreinigung und Thioredoxinfusion zur Erhöhung der Löslichkeit des exprimierten Enzyms, sowie pGEX mit Glutathionsynthetase-Fusion, sowie die pSPORT Vektoren. mit der Möglichkeit, die kodierende Region unterschiedlicher Vektoren des Gateway-Systems für verschiedene Expressionssysteme zu transferieren. Die Expressionsvektoren werden beispielsweise in λ DE3-lysogene *E. coli*-Stämme, z.B. BL21(DE3), HMS 174(DE3) oder AD494(DE3) transformiert. Nach oder während des Anwachsens der Zellen unter dem Fachmann geläufigen Standardbedingungen kann die Expression mit IPTG induziert werden. Nach Induktion der Zellen wird üblicherweise für 3 bis 24 Stunden bei Temperaturen von 18 bis 37°C inkubiert. Die Zellen können anschließend durch Sonifikation in Aufschlusspuffer (10 bis 200 mM Natriumphosphat, 100 bis 500 mM NaCl, pH 5 bis 8) oder Inkubation mit Lysispuffer aufgeschlossen werden. Das exprimierte Protein kann über Fällungen oder chromatographische Methoden gereinigt werden.

Die Erfindung umfasst weiterhin ein Verfahren zur Herstellung von Verbindungen der Formel (I) 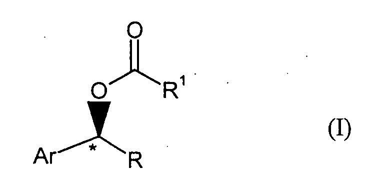 wobei Formel (I) die Absolutkonfiguration des Produktes angibt und in der
- *: ein stereogenes Kohlenstoffatom markiert und
- Ar: für C₅-C₁₄-Aryl steht und
- R: für Cyano, C₁-C₁₂-Alkyl, C₁-C₁₂-Halogenalkyl, C₅-C₁₁-Arylalkyl oder Reste der Formeln (IIa) bis (IIf) steht,

A-B-D (IIa)

A-D (IIb)

A-SO₂-R² (IIc)

A-SO₃W (IId)

A-COW (IIe)

A-N₃ (IIf)
in denen unabhängig voneinander
- A: fehlt oder für einen C₁-C₈-Alkylenrest steht und
- B: für eine Carbonyl-Gruppe steht und
- D: für R², OR², NHR³oder N(R³)₂ steht,
wobei R² für C₁-C₈-Alkyl, C₆-C₁₅-Arylalkyl, C₁-C₈-Halogenalkyl oder C₅-C₁₄-Aryl und
R³ jeweils unabhängig für C₁-C₈-Alkyl, C₆-C₁₅-Arylalkyl oder C₆-C₁₄-Aryl oder N(R³)₂ zusammen für einen cyclischen Aminorest steht und
W für OH, NH₂, oder OM steht, wobei M ein Alkalimetallion, ein halbes Äquivalent eines Erdalkalimetallions, ein Ammoniumion oder ein organisches Ammoniumion bedeuten kann und
- R¹: für C₁-C₁₂-Alkyl, C₁-C₁₂-Halogenalkyl, C₅-C₁₁-Arylalkyl, C₄-C₁₀-Aryl steht
das dadurch gekennzeichnet ist, dass
- Stereoisomerengemische von Verbindungen der Formel (III) 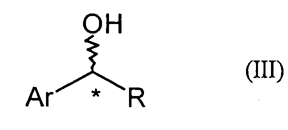 in der
   - *: Ar und R die unter der Formel (I) genannte Bedeutung besitzen
- mit Verbindungen der Formel (IV) 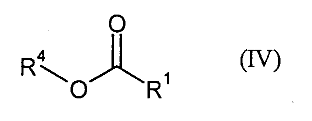 in der
   - R¹: die unter der Formel (I) genannte Bedeutung besitzt und
   - R⁴: für C₁-C₁₂-Alkyl, C₅-C₁₀-Aryl, C₆-C₁₁-Arylalkyl, C₂-C₁₂-Alkenyl oder C₁-C₁₂-Halogenalkyl steht
- in Gegenwart von erfindungsgemäßen Polypeptiden, die die biologische Aktivität einer Anti-Kazlauskas-Lipase ausüben,
umgesetzt werden.

Für die im erfindungsgemäßen Verfahren einsetzbaren Polypeptide gelten dabei die oben genannten Sequenzen, Vorzugsbereiche Parameter und Erläuterungen entsprechend. Der Begriff "in Gegenwart von erfindungsgemäßen Polypeptiden" umfasst dabei beispielsweise auch den Einsatz von rekombinanten Wirtszellen als solche oder gereinigte oder ungereinigte Zelllysate bzw. Zellpräparate.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Der Begriff "stereoisomerenangereichert" ("enantiomerenangereichert") im Sinne der Erfindung umfasst stereoisomerenreine (enantiomerenreine) Verbindungen oder beliebige Mischungen von stereoisomeren (enantiomeren) Verbindungen solange die stereoisomeren (enantiomeren) Verbindungen in diesen Mischungen nicht in gleichen relativen Anteilen (bzw. als Racemat) vorliegen.

Alkyl beziehungsweise Alkoxy bedeutet jeweils unabhängig einen geradkettigen, cyclischen, verzweigten oder unverzweigten Alkyl- beziehungsweise Alkylen- beziehungsweise Alkoxy-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₁₂-Alkyl weiter darüber hinaus beispielsweise für n-Nonyl, n-Decyl und n-Dodecyl.

C₁-C₄-Alkoxy steht beispielsweise für Methoxy, Ethoxy, n-Propoxy, iso-Propoxy, n-Butoxy, sec.-Butoxy und tert.-Butoxy, C₁-C₈-Alkoxy darüberhinaus für n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy, n-Hexoxy und n-Octoxy, C₁-C₁₂-Alkoxy weiter darüber hinaus beispielsweise für Adamantoxy, die isomeren Menthoxy-Reste, n-Decoxy und n-Dodecoxy.

Alkenyl steht im Rahmen der Erfindung jeweils unabhängig einen geradkettigen, cyclischen, verzweigten oder unverzweigten Alkenyl-Rest, der mindestens eine olefinische Doppelbindung aufweist und über ein olefinisches Kohlenstoffatom gebunden ist.

C₂-C₁₂-Alkenyl steht beispielsweise für Vinyl, 1-Propenyl, iso-Propenyl, 1-Butenyl, 2-Butenyl, 1-Pentenyl, 2-Pentenyl, 2-Methyl-1-butenyl, 2-Methyl-2-butenyl, 3-Methyl-1-buteny, 1-Hexenyl, 1-Heptenyl, 1-Octenyl oder 2-Octenyl.

Halogenalkyl steht im Rahmen der Erfindung jeweils unabhängig einen geradkettigen, cyclischen, verzweigten oder unverzweigten Alkyl-Rest, der durch Halogenatome einfach, mehrfach oder vollständig substituiert ist. Reste die vollständig durch Fluor substituiert sind, werden mit Perfluoralkyl bezeichnet.

Beispielsweise steht im Rahmen der Erfindung C₁-C₁₂-Halogenalkyl für Trifluormethyl, 2,2,2-Trifluorethyl, Chlormethyl, Fluormethyl, Brommethyl, 2-Bromethyl, 2-Chlorethyl, Nonafluorbutyl, n-Perfluoroctyl und n-Perfluordodecyl.

**Aryl und Ar** stehen im Rahmen der Erfindung beispielsweise für carbocyclische aromatische Reste mit 6 bis 14 Gerüstatomen wie vorzugsweise Phenyl, Naphthyl, Phenanthrenyl und Anthracenyl, oder für heteroaromatische Reste mit 5 bis 14 Gerüstatomen, in denen keines, ein, zwei oder drei Gerüstatome pro Cyclus, im gesamten Molekül mindestens jedoch ein Gerüstatom Heteroatome sind, die ausgewählt sind aus der Gruppe Stickstoff, Schwefel oder Sauerstoff wie vorzugsweise Pyridinyl, Thiophenyl, Benzofuranyl, Benzothiophen-yl, Dibenzofuran-yl, Dibenzothiophen-yl, Furanyl, Indolyl, Pyridazinyl, Pyrazinyl, Imidazolyl, Pyrimidinyl und Chinolinyl. Im Rahmen der Erfindung beziehen sich dabei Angaben wie z. B. C₅ im Falle von Arylresten auf die Anzahl der Kohlenstoffatome des aromatischen Gerüsts.

Weiterhin können die carbocyclischen aromatischen Reste oder heteroaromatischen Reste mit bis zu fünf gleichen oder verschiedenen Substituenten pro Cyclus substituiert sein. Beispielsweise und bevorzugt sind die Substituenten ausgewählt aus der Gruppe Fluor, Chlor, Nitro, Cyano, freies oder geschütztes Formyl, Hydroxy, C₁-C₁₂-Alkyl, C₁-C₁₂-Halogenalkyl, C₁-C₁₂-Alkoxy, C₁-C₁₂-Halogenalkoxy, C₅-C₁₀-Aryl wie zum Beispiel Phenyl, C₄-C₁₁-Arylalkyl wie zum Beispiel Benzyl, Di(C₁-C₁₂-alkyl)-amino, (C₁-C₁₂-Alkyl)-amino, CO(C₁-C₁₂-Alkyl), OCO(C₁-C₁₂-Alkyl), NHCO(C₁-C₁₂-Alkyl), N(C₁-C₆-Alkyl)CO(C₁-C₁₂-Alkyl), CO(C₃-C₁₂-Aryl), OCO(C₃-C₁₂-Aryl), NHCO(C₃-C₁₂-Aryl), N(C₁-C₆-Alkyl)CO(C₃-C₁₂-Aryl), COO-(C₁-C₁₂)-Alkyl, COO-(C₃-C₁₂)-Aryl, CON(C₁-C₁₂-Alkyl)₂ oder CONH(C₁-C₁₂-Alkyl) CO₂M, CONH₂, SO₂NH₂, SO₂N(C₁-C₁₂-Alkyl)₂, SO₃M wobei M jeweils für gegebenenfalls substituiertes Ammonium, Lithium, Natrium, Kalium oder Cäsium steht.

Analog gelten die Definition und die Vorzugsbereiche im Rahmen der Erfindung auch für den aromatischen Teil eines Arylalkyl-Restes.

Ar steht bevorzugt für Phenyl, Naphthyl, Pyridinyl, Oxazolyl, Thiophenyl, Furanyl, Benzofuranyl, Benzothiophen-yl, Dibenzofuran-yl, Dibenzothiophen-yl, Indolyl, Pyridazinyl, Pyrazinyl, Imidazolyl, Pyrimidinyl und Chinolinyl, die mit keinem, einem, zwei, drei oder vier Resten pro Cyclus weiter substituiert sein können, mit Resten die ausgewählt sind aus der Gruppe Hydroxy, Fluor, Chlor, Brom, Nitro, Cyano, C₁-C₈-Alkyl, C₁-C₈-Perfluoralkyl, C₁-C₈-Alkoxy, Di(C₁-C₄-alkyl)amino, COO(C₁-C₄-Alkyl), NHCO(C₁-C₄-Alkyl), CON(C₁-C₄-Alkyl)₂, COO(C₆-C₁₁-Arylalkyl), C₆-C₁₁-Arylalkyl oder C₅-C₁₀-Aryl.

Besonders bevorzugt steht Ar für Phenyl, mit keinem, einem oder zwei Resten substituiert sein kann, die ausgewählt sind aus der Gruppe Hydroxy, Fluor, Chlor, Brom, Methyl, Ethyl, iso-Propyl, Methoxy, Benzyloxy, Nitro, Cyano, Trifluormethyl, Phenyl, Dimethylamino und N-Acetylamino.

Ar steht ganz besonders bevorzugt für Phenyl und p-Chlorphenyl.
- R: steht bevorzugt für Methyl, Ethyl, Butyl, iso-Propyl, Benzyl, Chlormethyl, Brommethyl und Trifluormethyl.
- R¹: steht bevorzugt für Methyl, n-Butyl und iso-Propyl.
- R⁴: steht besonders bevorzugt für Ethyl, Vinyl, Isopropenyl, iso-Propyl und p-Chlorbenzoyl.

Besonders bevorzugte Verbindungen der Formel (III) sind p-Chlorbenzoylacetat, Ethylacetat, Isopropylbutyrat, Isopropylacetat und Isopropenylacetat, Trifluorethylbutyrat.

Als Stereoisomerengemische werden bevorzugt Enantiomerengemische von Verbindungen der Formel (II) eingesetzt. Das Verhältnis von (R)- zu (S)-konfigurierten Enantiomeren der Verbindungen der Formel (II) kann im Ausgangsgemisch beispielsweise 5:95 bis 95:5 betragen, bevorzugt 30:70 bis 70:30. Ganz besonders bevorzugt ist der Einsatz eines racemischen Gemisches.

Als Lösungsmittel für das erfindungsgemäße Verfahren sind beispielsweise geeignet: aliphatische und aromatische gegebenenfalls halogenierte Kohlenwasserstoffe wie beispielsweise Chlorbenzol, Xylol, Toluol, Benzol, Hexan und Dichlormethan.

Die Ausgangskonzentration an Verbindungen der Formel (II) kann beispielsweise zwischen 0,01 und 2 mol/l liegen, bevorzugt sind 0,1 bis 1 mol/l.

Die Temperatur bei der Reaktion kann beispielsweise 5 bis 120°C betragen, bevorzugt sind 20 bis 100°C, besonders bevorzugt 40 bis 80°C, besonders bevorzugt 60 bis 80°C.

Der Druck bei der Reaktion kann beispielsweise 0,01 bis 10 bar betragen, besonders bevorzugt 0,05 bis 1,2 bar und ganz besonders bevorzugt 0,05 bis 0,8 bar.

Durch den erniedrigten Druck können in einer bevorzugten Ausführungsform die meist flüchtigen Reaktionsprodukte aus den Verbindungen der Formel (III) aus dem Reaktionsgemisch destillativ entfernt werden.

Die Reaktionszeiten betragen beispielsweise 6 bis 96 Stunden, bevorzugt 12 bis 24 Stunden.

Auf erfindungsgemäße Weise wird beispielsweise bei der Umsetzung von racemischem Phenethylalkohol mit zunehmender Reaktionsdauer der (S)-konfigurierte Alkohol der Formel (II) zum (S)-konfigurierten Ester der Formel (II) umgesetzt, wobei sich der (R)-konfigurierte Alkohol der Formel (II) im Reaktionsgemisch anreichert.

Üblicherweise wird die Reaktion abgebrochen, wenn der Umsatz des ursprünglich eingesetzten Stereoisomerengemischs 60 bis 100 %, bevorzugt 80 bis 90 % des prozentualen Anteils an Alkohol der Formel (II) erreicht, das heißt bei racemischen Gemischen bei einem Umsatz von 30 bis 50 %, bevorzugt 40 bis 49 %.

Die Verbindungen der Formel (I) können dann in an sich bekannter Weise beispielsweise durch Destillation und/oder Chromatographie von den nicht umgesetzten Verbindungen der Formel (II) abgetrennt werden.

In einer besonders bevorzugten Ausführungsform wird das erfindungsgemäße Verfahrens jedoch in Gegenwart eines Katalysators durchgeführt, der stereoisomerenangereicherte Gemische von Verbindungen der Formel (II) racemisiert.

Auf diese Weise wird eine Anreicherung des nicht acylierten Alkohols der Formel (II) durch kontinuierliche Racemisierung weitgehend unterdrückt und letztendlich im optimalen Fall die gesamte eingesetzte Menge des Stereoisomerengemisches von Alkoholen der Formel (II) den Ester überführt. Auf diese Weise kann eine Trennung von Alkohol und Ester vermieden werden.

Bevorzugte Katalysatoren sind dabei solche, die Rutheniumkomplexe enthalten. Besonders bevorzugte Katalysatoren sind dabei solche, die
a) Rutheniumkomplexe der Formel (IV) enthalten, 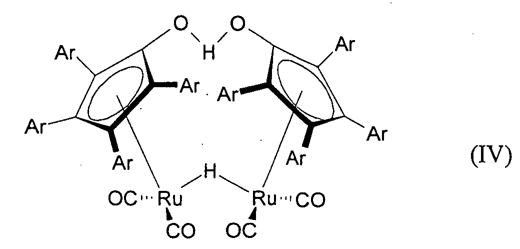 in der
   - Ar: jeweils unabhängig, bevorzugt identisch für Phenyl oder durch C₁-C₄-Alkylein-, zwei-, drei- oder vierfach substituiertes Phenyl steht, wobei Phenyl noch weiter bevorzugt ist,
   und/oder
b) Rutheniumkomplexe der Formel (V),

   [RuX₂(Aren)]₂ (V)

   in der
   - Aren: für eine koordinierte aromatische Verbindung mit 6 bis 12 Ringkohlenstoffatomen steht, die weiterhin mit bis zu 6 Resten substituiert sein kann, die jeweils unabhängig voneinander ausgewählt sind aus der Gruppe C₁-C₈-Alkyl, Benzyl und Phenyl und
   - X: für Chlor, Brom oder Iod, bevorzugt für Chlor steht
   und/oder
c) Rutheniumkomplexe der Formel (VI),

   [RuX₂(Aren){(VII)}] (VI)

   wobei
   - Aren und X: jeweils die unter Formel (V) angegebene Bedeutung besitzen und (VII) für sekundäre oder tertiäre Diamine, monoacylierte oder monosulfonierte Diamine, Aminoalkohole, Aminosäuren und Aminosäureamide steht.

In Formel (V) steht Aren bevorzugt für Benzol oder Naphthalin, das mit bis zu 6 Resten substituiert sein kann, die jeweils unabhängig voneinander ausgewählt sind aus der Gruppe Methyl, Ethyl, n-Propyl, Isopropyl und tert.-Butyl.

Bevorzugt steht Aren für Mesitylen, Cumöl oder Benzol.

Besonders bevorzugte Verbindungen der Formel (V) sind Benzoldichlororuthenium-Dimer, Mesitylendichlororuthenium-Dimer und Cumoldichlororuthenium-Dimer.

In Formel (VI) steht (VII) bevorzugt für Verbindungen der Formel (VII), 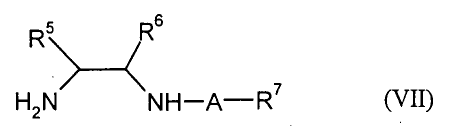 in der
- R⁵ und R⁶: jeweils unabhängig voneinander beispielsweise für Wasserstoff, C₁-C₂₀-Alkyl, C₄-C₁₅-Aryl oder C₅-C₁₆-Arylalkyl stehen oder R⁵ und R⁶ zusammen für einen geradkettigen oder verzweigten C₃-C₁₂-Alkylen-Rest stehen, und
- R⁷: für C₁-C₂₀-Alkyl, C₁-C₂₀-Fluoralkyl oder C₄-C₁₅-Aryl steht und
- A: für SO₂ oder CO, bevorzugt für SO₂ steht.

Bevorzugt stehen R⁵ und R⁶ jeweils identisch für Phenyl oder zusammen für geradkettiges C₃-C₈-Alkylen wie zum Beispiel 1,3-Propylen oder 1,4-Butylen, besonders bevorzugt stehen R⁷und R⁸ jeweils identisch für Phenyl.
- R⁷: steht bevorzugt für C₁-C₄-Alkyl, C₁-C₄-Fluoralkyl, Phenyl oder Naphthyl, das mit keinem, einem, zwei, drei vier oder fünf Resten weiter substituiert sein kann, die ausgewählt sind aus der Gruppe C₁-C₄-Alkyl, C₁-C₄-Alkoxy, C₁-C₄-Fluoralkyl, Fluor und Chlor, besonders bevorzugt für Methyl, Trifluormethyl, Pentafluorethyl, Nonafluorbutyl, Phenyl, p-Tolyl, p-Ethylphenyl, p-Anisyl, p-Ethoxyphenyl, p-Chlorphenyl, 2,4,6-Trimethylphenyl, 2,4,6-Triisopropylphenyl, p-Fluorphenyl, Pentafluorphenyl, und Naphthyl, wobei p-Tolyl, Phenyl und Naphtyl noch weiter bevorzugt sind.

Vorzugsweise erfolgt der einsatz des Katalysators in Gegenwart einer Base wie zum Beispiel Aminen des Typs NHₙ(C₁-C₁₈-Alkyl)₍₃₋ₙ₎, wobei n eins, zwei oder drei, bevorzugt drei sein kann. Einbesonders bevorzugtes Amin ist Triethylamin. Weiterhin können als Basen Alkali oder Erdalkalimetall -carbonate eingesetzt werden.

Die molare Menge an Ruthenium kann beispielsweise 0,01 bis 5 mol-%, bezogen auf das eingesetzte Substrat betragen, bevorzugt sind 0,1 bis 2 mol-%, ganz besonders bevorzugt 0,2 bis 1 mol-%.

Es ist vorteilhaft, wenn auch nicht zwingend notwendig, die Reaktion in einer sauerstofffreien Atmosphäre durchzuführen.

Auf erfindungsgemäße Weise können stereoisomerenangereicherte Ester der Formel (I) erhalten werden, die gegebenenfalls in an sich bekannter Weise zum Beispiel durch Destillation gereinigt werden können.

Weiterhin können die Verbindungen der Formel (I) durch Verseifung, die beispielsweise sauer oder basisch in an sich bekannter Weise erfolgen kann, in Verbindungen der Formel (IIa) überführt werden, 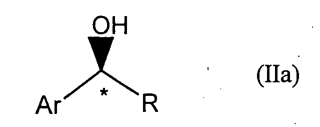 in der
- *, Ar und R: die unter der Formel (I) genannte Bedeutungen einschließlich deren Vorzugsbereiche besitzen.

Ein Verfahren zur Herstellung von Verbindungen der Formel (IIa) durch Hydrolyse von Stereoisomerengemischen von Verbindungen der Formel (Ia) 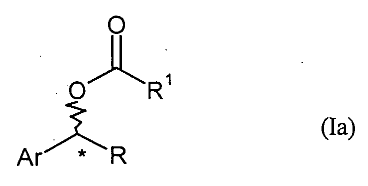 in der
- Ar, R und R¹: die unter der Formel (I) genannte Bedeutungen einschließlich deren Vorzugsbereiche besitzen und
- *: ein (S)- oder (R)-konfiguriertes Kohlenstoffatom markiert
in Gegenwart der erfindungsgemäßen Polypeptide ist von der Erfindung ebenfalls umfasst, stellt jedoch keine bevorzugte Herstellungsmethode dar.

Die erfindungsgemäß herstellbaren Verbindungen der Formeln (I) und (IIa) eignen sich insbesondere zur Anwendung in einem Verfahren zur Herstellung von Arzneimitteln oder Agrochemikalien sowie zur Herstellung von und als Zwischenprodukte von Arzneimitteln oder Agrochemikalien.

Der Vorteil der Erfindung liegt darin, dass durch den Einsatz der erfindungsgemäßen "Anti-Kazlauskas-Lipasen" aus einem Stereoisomerengemisch chiraler Alkohole selektiv das entgegengesetzte Enantiomer des entsprechenden Esters im Vergleich zu bekannten enzymatischen Racematspaltungen produziert werden kann.

Darüberhinaus gelingt erfindungsgemäß durch die Kombination der erfindungsgemäßen Lipasen mit einem Racemisierungskatalysator die Darstellung von enantiomerenangereicherten Estern mit sehr hohen Umsätzen und sehr guten Enantioselektivitäten. Weiterhin wird auch eine gute Enantioselektivität beobachtet, wenn der räumliche Anspruch der verschiedenen Substituenten R und S relativ gering ist, ein Vorteil gegenüber bisher bekannten Enzymen, die nur bei großen Unterschieden zwischen den Substituenten L und S hohe Enantioselektivitäten induzieren können.

### Beispiele:

### Beispiel 1

Es wurden insgesamt 275 Enzyme aus Genbanken in Umsetzungsexperimenten charakterisiert und auf ihre S-Selektivität gegenüber Stereoisomerengemischen von chiralen Alkoholen getestet.

Genbanken werden hergestellt, indem DNAs aus verschiedenen Umweltproben isoliert und anschließend in entsprechende Vektorsysteme kloniert werden. Diese werden in Wirtszellen wie z.B. *Escherichia coli* transformiert und exprimieren dort die heterologe DNA. Durch geeignete Screening-Verfahren können rekombinante Klone identifiziert werden, die die gewünschte Enzymaktivität aufweisen. Mit dieser Methode kann eine Vielzahl der in der Natur vorhandenen Enzyme zugänglich gemacht werden.

Um die Verwendungsmöglichkeit in der dynamisch kinetischen Racematspaltung zu prüfen, wurde die Veresterungsreaktion im organischen Lösungsmittel bei hohen Temperaturen durchgeführt.

Die rekombinanten *E. coli* Kulturen wurden durch dem Fachmann bekannte Methoden über Nacht bei 37 °C unter Zugabe von Induktor (IPTG) angezogen und am nächsten Morgen abzentrifugiert. Zur Lyse der Zellen werden die Zellpellets in Lysispuffer (20 mM Ammoniumformiat pH 6,5, 0,5% Lysozym, 0,5% Dodecyl β-D Maltosid) resuspendiert und 3 bis 4 Stunden bei 37 ⁰C unter Schütteln inkubiert. Grobe Zelltrümmer werden anschliessend durch erneute Zentrifugation abgetrennt und der Überstand Lyophilisiert. Die Lyophilisate der Zellen werden als Katalysator für die Umsetzungen eingesetzt.

Die Reaktionen erfolgten in 1 ml Toluol (getrocknet) unter folgenden Reaktionsbedingungen:
- eine Spatelspitze Lyophilisat
- 0,2 M Substrat
- 0,6 M Vinylacetat
- 4 Stunden bei 60°C bzw. 80°C

Nach erfolgter Reaktion werden die Proben mit chiraler GC gemessen und die Ergebnisse analysiert.

Es konnte bei den Versuchen eine Anti-Kazlauskas-Lipase mit der Aminosäuresequenz gemäß SEQ ID NO: 2 identifiziert werden.

### Allg. Arbeitsvorschrift für die Racematspaltung von Phenylalkanolen:

In einem Reaktionsrohr wird die Anti-Kazlauskas-Lipase in 4 ml Toluol vorgelegt, das Gefäß mit Argon gespült und der Alkohol, das Acylierungsmittel und gegebenenfalls der Racemisierungskatalysator zugesetzt. Es wird auf die gewünschte Temperatur aufgeheizt und für die angegebene Zeit gerührt.

Die Ergebnisse der durch die Anti-Kazlauskas-Lipase katalysierten Acylierung werden in Tabelle 1 dargestellt.

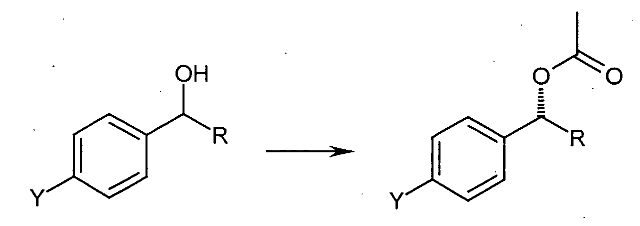

**Tabelle 1:**

| Beispiel | Lyophilisat [Gew. %] | Acylierungsmittel | Y | R | Kat. | T [°C] | t [h] | U [%] | ee (Ester[%]) |
|---|---|---|---|---|---|---|---|---|---|
| 2 | 10 | VAc | H | Me | - | 80 | 16 | 11,7 | 44,4 (S) |
| 3 | 10 | VAc | H | Me | - | 80 | 40 | 16,3 | 38 (S) |
| 4 | 10 | ClBA | H | Me | - | 80 | 40 | 11,6 | 45 (S) |
| 5 | 10 | ClBA | H | Me | + | 80 | 16 | 27,3 | 34 (S) |
| 6 | 5 | ClBA | H | Et | + | 80 | 16 | 24 | n.d. |
| 7 | 5 | ClBA | H | Et | + | 80 | 40 | 31,9 | 41,8 (S) |
| 8 | 5 | ClBA | H | Et | - | 80 | 40 | 3,9 | 56,6 (S) |
| 9 | 10 | ClBA | H | i-Pr | + | 80 | 40 | 14,7 | 80,4 (S) |
| 10 | 10 | ClBA | Cl | Me | - | 80 | 80 | 3,0 | 45 (S) |
| 11 (z. Vgl.) | 10 | ClBA | H | | + | 80 | 40 | <1 | 0 |
| Erläuterungen zur Tabelle | | | | | | | | | |
| Acylierungsmittel: | | | | | | | | | |
| Vac = Vinylacetat (3 eq), ClBA = Chlorbenzoylacetat (3 eq), | | | | | | | | | |
| Kat. = Katalysator (je 2 mol % [(C₄Ph₄COHOCC₄Ph₄)(µ-H)][(CO)₄Ru₂] | | | | | | | | | |
| z. Vgl: Vergleichsbeispiel: Enzym Candida Antarctica 435 (Fa. Novo Nordisk). | | | | | | | | | |

## Patentansprüche

1. Nukleinsäuren die für Polypeptide codieren, die die biologische Aktivität einer Anti-Kazlauskas-Lipase ausüben und eine Aminosäuresequenz gemäß SEQ ID NO: 2 umfassen.

2. Nukleinsäuren gemäß Anspruch 1, **dadurch gekennzeichnet, dass** es sich um einzelsträngige oder doppelsträngige Desoxyribonukleinsäuren (DNA) oder Ribonukleinsäuren (RNA) handelt.

3. Nukleinsäuren gemäß mindestens einem der Ansprüche 1 bis 2, **dadurch gekennzeichnet, dass** es sich um Fragmente genomischer DNA oder cDNA handelt.

4. Nukleinsäuren gemäß mindestens einem der Ansprüche 1 bis 3, umfassend eine Sequenz ausgewählt aus
a) der Sequenz gemäß SEQ ID NO: 1,
b) Sequenzen, die für ein Polypeptid codieren, welches die Aminosäuresequenz gemäß SEQ ID NO: 2 umfasst,
c) zumindest 14 Basenpaare langen Teilsequenzen der unter a) oder b) definierten Sequenzen,
d) Sequenzen, welche an die unter a) oder b) definierten Sequenzen hybridisieren,
e) Sequenzen, welche eine zumindest 70 %ige Identität mit den unter a) definierten Sequenzen aufweisen,
f) Sequenzen, welche eine zumindest 70 %ige Identität mit den unter b) definierten Sequenzen aufweisen,
g) Sequenzen, welche zu den unter a) oder b) definierten Sequenzen komplementär sind, und
h) Sequenzen, welche aufgrund der Degeneriertheit des genetischen Codes für dieselbe Aminosäuresequenz codieren wie die unter a) bis f) definierten Sequenzen.

5. DNA-Kontrukt umfassend eine Nukleinsäure gemäß mindestens einem der Ansprüche 1 bis 4 und einen heterologen Promotor.

6. Vektor umfassend eine Nukleinsäure gemäß mindestens einem der Ansprüche 1 bis 4 oder ein DNA-Konstrukt gemäß Anspruch 5 .

7. Wirtszelle enthaltend eine Nukleinsäure gemäß mindestens einem der Ansprüche 1 bis 4, ein DNA-Konstrukt gemäß Anspruch 5 oder einen Vektor gemäß Anspruch 6.

8. Wirtszelle gemäß Anspruch 7, **dadurch gekennzeichnet, dass** es sich um eine prokaryotische Zelle handelt.

9. Verfahren zur Herstellung einer Nukleinsäure gemäß mindestens einem der Ansprüche 1 bis 4, **dadurch gekennzeichnet, dass** die Nukleinsäure
• chemisch synthetisiert wird oder
•
a) Oligonukleotide chemisch synthetisiert werden,
b) die Oligonukleotide radioaktiv oder mit einem Fluoreszenzfarbstoff markiert werden,
c) die markierten Oligonukleotide an DNA einer genomischen oder cDNA-Bank, die ausgehend von Pflanzen-mRNA oder genomischer DNA hergestellt wurde, hybridisiert wird,
d) Klone selektiert werden, an die die markierten Oliogonukleotide hybridisieren und
e) Die hybridisierte DNA isoliert wird oder
• Oligonukleotide chemische synthetisiert werden und mittels PCR amplifiziert werden.

10. Polypeptid mit der biologischen Aktivität einer Anti-Kazlauskas-Lipase, welches von einer Nukleinsäure gemäß mindestens einem der Ansprüche 1 bis 4 codiert wird.

11. Polypeptid mit der biologischen Aktivität einer Anti-Kazlauskas-Lipase, welches eine Aminosäuresequenz umfasst, die eine zumindest 60%-ige, Identität mit einer Aminosäuresequenz gemäß SEQ ID NO: 2 über zumindest den Abschnitt von Aminosäure 110 bis 280 Aufweist.

12. Verfahren zur Herstellung eines Polypeptids gemäß mindestens einem der Ansprüche 10 bis 11, **dadurch gekennzeichnet dass**
• die Herstellung durch chemische Methoden erfolgt oder
• Wirtszellen gemäß mindestens einem der Ansprüche 7 bis 8 unter Bedingungen angezogen werden, die die Expression einer Nukleinsäure gemäß mindestens einem der Ansprüche 1 bis 4 erlaubt, die angezogenen Wirtszellen geerntet werden und das Polypeptid in an sich bekannter Weise daraus gewonnen und gegebenenfalls gereinigt wird.

13. Verfahren zur Herstellung von Verbindungen der Formel (I) 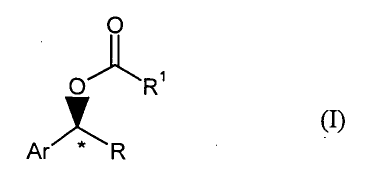 wobei Formel (I) die Absolutkonfiguration des Produktes angibt und in der
* ein stereogenes Kohlenstoffatom markiert und
Ar für C₅-C₁₄-Aryl steht und
R für Cyano, C₁-C₁₂-Alkyl, C₁-C₁₂-Halogenalkyl, C₅-C₁₁-Arylalkyl oder Reste der Formeln (IIa) bis (IIf) steht,
A-B-D (IIa)
A-D (IIb)
A-SO₂-R³ (IIc)
A-SO₃W (IId)
A-COW (IIe)
A-N₃ (IIf)
in denen unabhängig voneinander
A fehlt oder für einen C₁-C₈-Alkylenrest steht und
B für eine Carbonyl-Gruppe steht und
D für R², OR², NHR³oder N(R³)₂ steht,
wobei R² für C₁-C₈-Alkyl, C₆-C₁₅-Arylalkyl, C₁-C₈-Halogenalkyl oder C₅-C₁₄-Aryl und
R³ jeweils unabhängig für C₁-C₈-Alkyl, C₆-C₁₅-Arylalkyl oder C₆-C₁₄-Aryl oder N(R³)₂ zusammen für einen cyclischen Aminorest steht und
W für OH, NH₂, oder OM steht, wobei M ein Alkalimetallion, ein halbes Äquivalent eines Erdalkalimetallions, ein Ammoniumion oder ein organisches Ammoniumion bedeuten kann und
R¹ für C₁-C₁₂-Alkyl, C₁-C₁₂-Halogenalkyl, C₅-C₁₁-Arylalkyl, C₄-C₁₀-Aryl steht
das **dadurch gekennzeichnet ist, dass**
Stereoisomerengemische von Verbindungen der Formel (III) 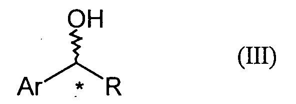 in der
*, Ar und R die unter der Formel (I) genannte Bedeutung besitzen
• mit Verbindungen der Formel (IV) 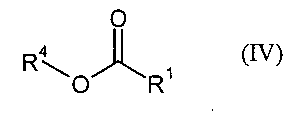 in der
R¹ die unter der Formel (I) genannte Bedeutung besitzt und
R⁴ für C₁-C₁₂-Alkyl, C₄-C₁₀-Aryl, C₅-C₁₁-Arylalkyl, C₂-C₈-Alkenyl oder C₁-C₁₂-Halogenalkyl steht
• in Gegenwart von erfindungsgemäßen Polypeptiden, die die biologische Aktivität einer Anti-Kazlauskas-Lipase ausüben,
umgesetzt werden.

14. Verfahren nach Anspruch 13, **dadurch gekennzeichnet, dass** Ar für Phenyl, Naphthyl, Pyridinyl, Oxazolyl, Thiophenyl, Furanyl, Benzofuranyl, Benzothiophen-yl, Dibenzofuran-yl, Dibenzothiophen-yl, Indolyl, Pyridazinyl, Pyrazinyl, Imidazolyl, Pyrimidinyl oder Chinolinyl steht, das mit keinem, einem, zwei, drei oder vier Resten pro Cyclus weiter substituiert sein kann, mit Resten die ausgewählt sind aus der Gruppe Hydroxy, Fluor, Chlor, Brom, Nitro, Cyano, C₁-C₈-Alkyl, C₁-C₈-Perfluoralkyl, C₁-C₈-Alkoxy, Di(C₁-C₄-alkyl)amino, COO(C₁-C₄-Alkyl), NHCO(C₁-C₄-Alkyl), CON(C₁-C₄-Alkyl)₂, COO(C₆-C₁₁-Arylalkyl), C₆-C₁₁-Arylalkyl oder C₅-C₁₀-Aryl.

15. Verfahren gemäß mindestens einem der Ansprüche 13 und 14, **dadurch gekennzeichnet, dass** als Verbindung der Formel (III) p-Chlorbenzoylacetat, Ethylacetat, Isopropylbutyrat, Isopropylacetat, Isopropenylacetat oder Trifluorethylbutyrat eingesetzt wird.

16. Verfahren gemäß mindestens einem der Ansprüche 13 bis 15, **dadurch gekennzeichnet, dass** als Stereoisomerengemische racemische Gemische von Verbindungen der Formel (II) eingesetzt werden.

17. Verfahren gemäß mindestens einem der Ansprüche 13 bis 16, **dadurch gekennzeichnet, dass** die Reaktion abgebrochen wird, wenn der Umsatz des ursprünglich eingesetzten Stereoisomerengemischs 60 bis 100 % des prozentualen Anteils an (S)-konfiguriertem Alkohol der Formel (II) erreicht.

18. Verfahren gemäß mindestens einem der Ansprüche 13 bis 16, **dadurch gekennzeichnet, dass** das Verfahren in Gegenwart eines Katalysators durchgeführt wird, der stereoisomerenangereicherte Gemische von Verbindungen der Formel (II) racemisiert.

19. Verfahren gemäß Anspruch 18, **dadurch gekennzeichnet, dass** als Katalysoren solche eingesetzt werden, die Rutheniumkomplexe enthalten.

20. Verfahren gemäß Anspruch 18, **dadurch gekennzeichnet, dass** als Katalysoren solche eingesetzt werden, die
a) Rutheniumkomplexe der Formel (IV) enthalten, 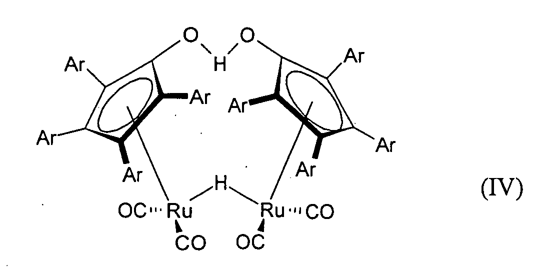 in der
Ar jeweils unabhängig für Phenyl oder durch C₁-C₄-Alkyl- ein-, zwei-, drei- oder vierfach substituiertes Phenyl steht,
und/oder
b) Rutheniumkomplexe der Formel (V),
[RuX₂(Aren)]₂ (V)
in der
Aren für eine koordinierte aromatische Verbindung mit 6 bis 12 Ringkohlenstoffatomen steht, die weiterhin mit bis zu 6 Resten substituiert sein kann, die jeweils unabhängig voneinander ausgewählt sind aus der Gruppe C₁-C₈-Alkyl, Benzyl und Phenyl und
X für Chlor, Brom oder Iod, bevorzugt für Chlor steht
und/oder
c) Rutheniumkomplexe der Formel (VI),
[RuX₂(Aren){(VII)}] (VI)
wobei
Aren und X jeweils die unter Formel (V) angegebene Bedeutung besitzen und (VII) für sekundäre oder tertiäre Diamine, monoacylierte oder monosulfonierte Diamine, Aminoalkohole, Aminosäuren und Aminosäureamide steht.

21. Verfahren gemäß Anspruch 20, **dadurch gekennzeichnet, dass** in Formel (VI) (VII) für Verbindungen der Formel (VII) steht, 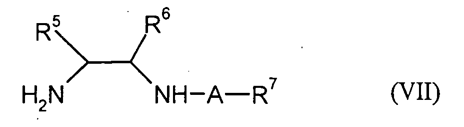 in der
R⁵ und R⁶ jeweils unabhängig voneinander beispielsweise für Wasserstoff, C₁-C₂₀-Alkyl, C₄-C₁₅-Aryl oder C₅-C₁₆-Arylalkyl stehen oder R⁵ und R⁶ zusammen für einen geradkettigen oder verzweigten C₃-C₁₂-Alkylen-Rest stehen, und
R⁷ für C₁-C₂₀-Alkyl, C₁-C₂₀-Fluoralkyl oder C₄-C₁₅-Aryl steht und
A für SO₂ oder CO steht.

22. Verfahren gemäß mindestens einem der Ansprüche 18 bis 21, **dadurch gekennzeichnet, dass** der Einsatz des Katalysators in Gegenwart einer Base erfolgt.

23. Verfahren gemäß mindestens einem der Ansprüche 18 bis 22, **dadurch gekennzeichnet, dass** in einem weiteren Schritt die Verbindungen der Formel (I) durch Verseifung in Verbindungen der Formel (IIa) überführt werden, 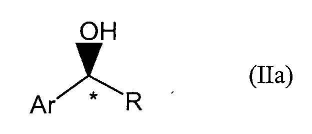 in der
*, Ar und R die unter der Formel (I) in Anspruch 18 genannte Bedeutungen besitzen.

24. Verfahren zur Herstellung von Verbindungen der Formel (IIa) 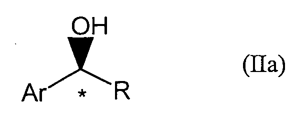 in der
Ar und R die unter der Formel (I) in Anspruch 13 genannte Bedeutungen besitzen und
* ein (S)-stereogenes Kohlenstoffatom in seiner Absolutkonfiguration markiert,
**dadurch gekennzeichnet, dass**
Stereoisomerengemische von Verbindungen der Formel (Ia) 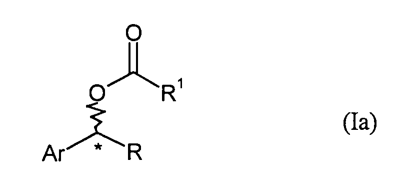 in der
Ar, R und R¹ die unter der Formel (I) genannten Bedeutungen besitzen und
* ein (S)-stereogenes Kohlenstoffatom markiert
in Gegenwart von Polypeptiden gemäß mindestens einem der Ansprüche 10 bis 11 hydrolysiert werden.

25. Verwendung von Verbindungen, die gemäß mindestens einem der Ansprüche 13 bis 23 oder Anspruch 24 hergestellt wurden in einem Verfahren zur Herstellung von Arzneimitteln oder Agrochemikalien oder als Zwischenprodukte von Arzneimitteln oder Agrochemikalien.


### Sequenzprotokoll

<110> Bayer Aktiengesellschaft
   <120> Anti-Kazlauskas-Lipasen
   <130> LeA 35 991
   <160> 2
   <170> PatentIn version 3.1
<210> 1
   <211> 885
   <212> DNA
   <213> artifiziell
   <220>
   <221> CDS
   <222> (1)..(885)
   <223>
<400> 1 
atg gca cag gtg aag gcc aac ggc att acc ctc gag tat gaa gag cag
48
Met Ala Gln Val Lys Ala Asn Gly Ile Thr Leu Glu Tyr Glu Glu Gln
1               5                   10                  15

ggc cat cgc cac cat ccg tcc atg ctc ctc att atg ggc ctg ggc ggc
96
Gly His Arg His His Pro Ser Met Leu Leu Ile Met Gly Leu Gly Gly
            20                  25                  30

cag tta atc gac tgg ccc gag gag ttc atc cgg ggg ctg gct gaa cga
144
Gln Leu Ile Asp Trp Pro Glu Glu Phe Ile Arg Gly Leu Ala Glu Arg
        35                  40                  45

ggc ttc cgg gta atc tgt ttc gac aac cgc gac gcg ggg ctt tcg acg
192
Gly Phe Arg Val Ile Cys Phe Asp Asn Arg Asp Ala Gly Leu Ser Thr
    50                  55                  60

aaa ctt gaa ggc gtg aaa aaa ccg aac att gcc cgg gta ttt ctc ctg
240
Lys Leu Glu Gly Val Lys Lys Pro Asn Ile Ala Arg Val Phe Leu Leu
65                  70                  75                  80

gcg agc atg ggc cta aag ccc agg gtg cct tac acc ctc gac gac atg
288
Ala Ser Met Gly Leu Lys Pro Arg Val Pro Tyr Thr Leu Asp Asp Met
                85                  90                  95

gcc ctg gac acc gtg ggg ctg atg gat gcc ctg ggc att gag agc acc
336
Ala Leu Asp Thr Val Gly Leu Met Asp Ala Leu Gly Ile Glu Ser Thr
            100                 105                 110

cac gta gtt ggc gtc tcc atg ggc ggc atg att gcg cag att cta ggg
384
His Val Val Gly Val Ser Met Gly Gly Met Ile Ala Gln Ile Leu Gly
        115                 120                 125

gcg aag cac ggg gag cgg gtg aaa tcc ctt acc ctg atg att acc tcc
432
Ala Lys His Gly Glu Arg Val Lys Ser Leu Thr Leu Met Ile Thr Ser
    130                 135                 140
 
tcc ggc aac ccc cgc atg ccg gcg ccc agg ccg cag gtg ctg caa aag
480
Ser Gly Asn Pro Arg Met Pro Ala Pro Arg Pro Gln Val Leu Gln Lys
145                 150                 155                 160

ttt atg cgg gtg ccc aag agc atg gat aag gaa gag tgg att aaa tac
528
Phe Met Arg Val Pro Lys Ser Met Asp Lys Glu Glu Trp Ile Lys Tyr
                165                 170                 175

aac ttg gag ctt tta acc acc atc ggc agc ccc ggg ttg gac cgg gag
576
Asn Leu Glu Leu Leu Thr Thr Ile Gly Ser Pro Gly Leu Asp Arg Glu
            180                 185                 190

aag ctg gcc tta gac gtg agg aag agc ata gag cgg tgc ctt tgc ccc
624
Lys Leu Ala Leu Asp Val Arg Lys Ser Ile Glu Arg Cys Leu Cys Pro
        195                 200                 205

gaa ggc acg cag cgg cag ctg gca gcc atc ctg cag agc ggc agc agg
672
Glu Gly Thr Gln Arg Gln Leu Ala Ala Ile Leu Gln Ser Gly Ser Arg
    210                 215                 220

gtg aag ctg ctc cgg cgg atc gct gtc ccc acc ctg gtc atc agc ggg
720
Val Lys Leu Leu Arg Arg Ile Ala Val Pro Thr Leu Val Ile Ser Gly
225                 230                 235                 240

gcg gaa gat ccc ctc ctg ccg tac cag tgc ggc cgg gac att gcc gac
768
Ala Glu Asp Pro Leu Leu Pro Tyr Gln Cys Gly Arg Asp Ile Ala Asp
                245                 250                 255

cat atc ccg gga gcc cgc ttc gag ctc atc gag ggc atg ggg cac gac
816
His Ile Pro Gly Ala Arg Phe Glu Leu Ile Glu Gly Met Gly His Asp
            260                 265                 270

att ccc gag cgg cac atc ccc cgg ctg att gag ctc atc gcc ggg cac
864
Ile Pro Glu Arg His Ile Pro Arg Leu Ile Glu Leu Ile Ala Gly His
        275                 280                 285

gcc         gcg         gcc         gcg         gaa         gct         taa
885
Ala Ala Ala Ala Glu Ala
    290
 <210> 2
   <211> 294
   <212> PRT
   <213> artifiziell
<400> 2